# EUROPEAN PATENT APPLICATION

(11) **EP 0 754 462 A2**
(43) Date of publication of application: **22.01.1997**
(21) Application number: 96110682.0
(22) Date of filing: 02.07.1996
(51) Int. Cl.: A61K 35/407

(54) **Auxiliary liver with long term storability and production thereof**

(30) Priority: 19.07.1995 JP 182658/95
(71) Applicant: TTI Co., Ltd., Meguro-ku, Tokyo 153 (JP)
(72) Inventor: Tsuji, Kimiyoshi, Yokohama-shi, Kanagawa-pref., 241 (JP)
(74) Representative: Flach, Dieter Rolf Paul, Dipl.-Phys.

(57) **Abstract**

An auxiliary liver and the related product suitable for implantation, the liver obtained from a fetal pig and treated with an antifreeze solution that allows for the long term storage with retention of biological function.

## Description

This invention relates to the production of an auxiliary liver suitable for long term storage, which utilizes liver fragments from a porcine fetus, and to a product using the fragments suitable for implantation in a human patient.

There are two approaches for the treatment of liver dysfunctions, and the impairment and loss of liver functions, *i.e.*, surgical and non-surgical measures. Non-surgical treatment consists of resting, relaxing, diet, drugs, biological preparations such as interferon, etc., however, they do not radically treat the liver dysfunctions. Surgical treatment includes dissection for therapeutic purpose and due to trauma, plasma exchange, organ transplant and replacement with total or hybrid artificial livers. There are no effective methods by means of surgical procedures.

Liver transplants follow two approaches: cell or tissue transplantation and transplanting the whole liver. At the present, the former approach is limited by the difficulty in preserving biological functions of cells and tissues for a long time. The latter may be divided into allo and xeno liver transplants each of which has their own inherent problems. An allo transplant is preferred for a better result, but securing a sufficient number of related donors is difficult. Even in the case of an allo transplant, the effect of immunosuppressants does not totally alleviate the possibility of rejection by the recipient, who receives an immuno-incompatible organ. In such a case the life of the recipient may not be saved. While the immuno-compatibility of a transplanted organ is important for success, it is not always possible to assure the availability of a perfectly matched liver for transplantation. In the case of a xeno transplant, the use of liver from a pig and a baboon has been reported with only a temporary prolongation of life.

Presently available remedies for the treatment of liver dysfunctions are not satisfactory as described above and a new method for treating liver dysfunctions particularly in cases of emergency, and possibly for a longer term effect, is badly needed, The present invention offers at auxiliary liver for all types of the liver dysfunctions and a method of producing an auxiliary liver having a long term storability.

An auxiliary liver product suitable for long term storage according to this invention is made from the liver of a pig fetus at a gestational age over about 70 days, and preferably over about 90 days. The fetus is aseptically removed from the pregnant pig and the aseptically recovered fetal liver is dissected into small fragments and stored at -198°C to preserve their biological activity. The liver fragments are stored in anti-freeze or preserving solution by placing the fetal liver. A solution containing 5.0-10.0% dimethyl sulfoxide (DMSO) or glycerol in a 40.0-60.0% phosphate buffer may be used as an anti-freeze solution. A nutrient media such as L-15 (Gibeo) also may be added to the anti-freeze solution. For the purpose of this invention, the fetal liver fragments are produced having a maximum dimension of about 0.5-5.0 mm, preferably about 1.0-3.0 mm and most preferably in cube form.

The process of making the auxiliary liver product with a long term storability comprises aseptically removing a fetus at a gestational age of 70 days from a pregnant pig, removing the liver from the fetus, dissecting the fetal liver into fragments (hereinafter referred to as fetal liver fragments or FLF), treating them such as by immersing in an anti-freeze solution and then storing the treated FLF at 198°C whereby the FLF are protected from loss of biological activities. Ultimately, the FLF are placed in a capsule for implantation in a mammal, where the capsule is made of a membrane with selective permeability.

Immuno-incompatibility between different animals occurs due to the major histocompatibility complex (MHC). In this invention, by using immunogenically immature porcine fetal liver tissue one minimizes the immunological incompatibility due to MHC; thus, minimizing the chance of rejection by the recipient. Therefore, it is possible to achieve a life saving effect heretofore not possible with conventional technology and the invention makes it possible to use the liver from animals, and especially pigs, in humans.

Unlike cells, the bioactivity of tissues normally can not be maintained by the usual method of freezing or refrigeration. When tissues are kept by freezing, it is thawed at the time of use, Unfortunately, the bioactivity of the tissue is lost, or at least severely reduced by conventional freezing and thawing. It is a discovery of this invention that the pig fetal liver when cut into fragments (FLF) allows an antifreeze solution, containing a suitable preservative such as DMSO or glycerol, to penetrate into the tissue and makes it possible to freeze all parts of the FLF tissue uniformly. In this way, long term storage of the FLF becomes possible with the maintenance (retention) of its bioactivity.

The antifreeze solution used in this invention apparently allows the transfer of water between tissue cells and their surrounding environment. Therefore, water, which normally is otherwise trapped in the cells is removed from the cells and does not break up the cell walls by expansion at the time of freezing, In this way, the FLF tissues may be stored by freezing without destroying their bioactivity.

For ultimate use in a patient, the FLF are occluded or encapsulated within a permeable membrane for transplantation. The membrane to be used for making the capsules or containers (sachet) must have a specified size of pores so that when they are inserted into an animal patient (recipient), the membrane blocks out larger molecules such as antigens and other components associated with an immune response from the recipient, but the membrane allows smaller molecules, such as the material to be metabolized by the FLF, to reach the fetal liver tissues. The membrane thus stops host antigens from attacking the FLF and passes metabolites through it. In this way, the bioactivity of FLF is protected when placed in a different animal.

These as well as other objects of the present invention will become apparent during the course of the following description with reference to the accompanying drawings, in which:
Figure 1 is an overhead view of an implantable product of this invention.
Figure 2 is a front view of a procedure for making the sachet for the FLF product using a semi-permeable membrane.
Figure 3 is the side view of the procedure for making the sachet or capsule for the FLF product from the semi-permeable membrane.

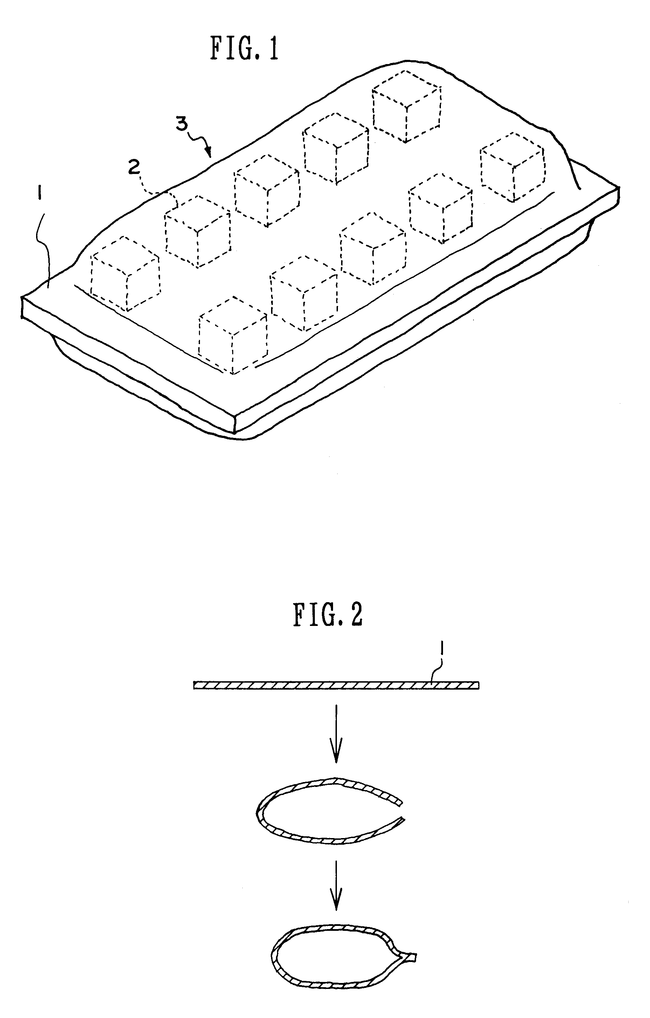

Referring now to the accompanying drawings, an example of a process of making an implantation product using porcine fetal liver fragments (PFLF) is shown in the attached Figures 1 through 3.

In the methods, specific pathogen free (SPF) pigs are used. Landrace and Birkshire species of pig have been used in the examples, but other species may also be used for the purpose of the invention.

The fetus, at a gestational age of over 70 days, is removed from its mother pig by a cesarean operation and the liver is taken out from the fetus. Usually, a mother produces 8 to 12 fetuses. Infection of the fetus by viruses, bacteria, spirochetes, parasites, and other pathogenic organisms may be determined by gene detection, antibody testing, and serological methods. The selection of the gestational age over 70 days is chosen so as to avoid harvesting premature tissues. The size of the liver normally is too small and the liver generally is too immature before 70 days, which may cause the PFLF to have an insufficient bioactivity.

The fetal liver is washed as soon after the removal as is practical using, for example, a phosphate buffer solution containing an antibiotic and the liver is kept at 40°C. The liver is cut, preferably into 1-3 mm pieces, by a laser knife, for example. For removing blood cells in the tissue, FLF is washed with phosphate buffer solution containing 0.54 mM EDTA and then with a 20% phosphate buffer solution. The average size of a porcine fetal liver is 40-50 mm in diameter and 20 mm in thickness. A pig fetus, therefore, typically yields about 800-1,300 pieces of about 3 mm each.

FLF are immersed in a preserving solution for protecting the tissue from damage by freezing. A suitable aqueous preserving solution is a 50% phosphate buffer containing 7.5% DMSO kept at 4°C. L-15 nutrient solution may be added to the preserving solution. When required, about 5 microlitters of the preserving solution is injected into FLF 2-3 times. The penetration of the preserving solution may be improved by washing FLF with phosphate buffer solution containing EDTA before the injection. In one embodiment for storing the FLF, 10-15 pieces each of FLF, injected with the preserving solution using a hypodermic needle, are placed in freezing tubes with an additional 1 ml of the preserving solution. These processes are carried out at 4°C. FLF then is frozen, first to -80°C in a programmed freezer in 1°C steps and eventually to -198°C in liquid nitrogen for storage. Any suitable container for use at liquid nitrogen temperature can be used for storing the FLF.

An alternative method for injecting the preserving solution into or imbibing the preserving solution in the FLF is to directly immerse the FLF in the preserving solution. While more care must be observed to obtain a sufficient penetration of the preserving solution by simple immersion, such process is more convenient to use than the injection of the solution directly into the tissue. With care, it is possible to achieve the desired penetration by immersion, particularly if the FLF is made at the smaller size range. A suitable immersion time can be determined by routine experimentation.

Usually, as soon as liver is cut into fragments, the FLF are placed in the antifreeze solution. An antifreeze solution bath should be provided sufficient to cover all the fragments. This procedure is carried out at 4°C. When the preparation of fragments is finished, the mixture of fragments and the immersions solution is placed in a programmed freezer and the freezing process starts to lower the temperature by 1°C per minute. Thus about 84 minutes is necessary to reach -80°C, and the FLF remains immersed for that whole period.

FLF prepared by the aforementioned processed may be kept frozen for a prolonged period in liquid nitrogen. At the time of use, the frozen FLF is thawed quickly at 37°C. The bioactivity of FLF can be tested by ordinary and specific staining for microscopic observation *in vitro* and by placing it in the omentum of a dog for biochemical and histological observations *in vivo*. When using this invention, it has been observed that the bioactivity of FLF after thawing is nearly 100% of the original activity in many cases.

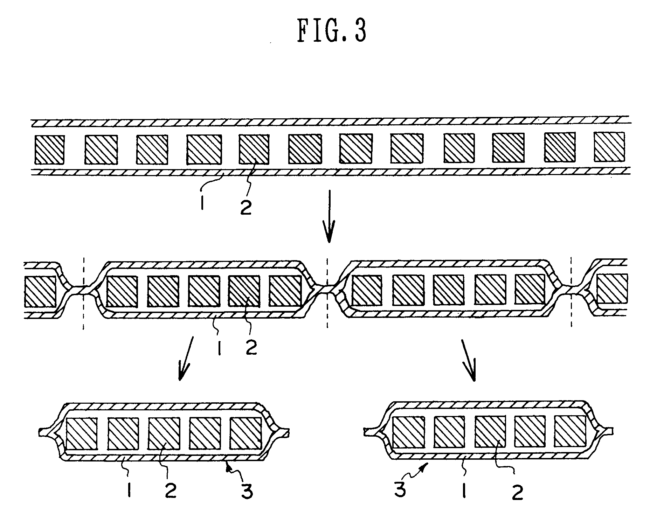

FLF quickly thawed at 37°C is washed twice with 20% phosphate buffer solution containing L-15 nutrient solution. The thawed FLF are then used to prepare an implantation product. A membrane is cut, for example into a 30 mm wide strip, which is made into a tube by folding longitudinally and by sealing the edge with an impulse sealer (Figure 2). The washed FLF is placed in the tube, which is sealed longitudinally at every 20 mm with at impulse sealer. The tube can be cut at the seal to form an auxiliary liver product (Figure 3) suitable for implantation. The product has dimensions of about 10x20x3 mm containing about 10 FLF in each package. FLF to be used may be a mixture of FLF from several different fetuses. It is not necessary to line up FLF in the tube and they may be placed in the package at random.

The membrane should have a permeability which stops the flux of the larger host antigen (immune) molecules, but permits substances which should be metabolized by the host liver to pass. For example, a multiporous polypropylene film with the pore size of about 0.075 x 0.25 micrometer and a thickness of 25 micrometers as supplied by Senko Ika Co. may be used. A membrane supplied by Hoechst under the registered trademark "Celgard" 2500 having an effective pore size of 0.075 micrometer may also be used. The capsule made of the selectively permeable membrane in this invention is not limited to the design as described above. It may be made into other forms (wrapped, sachet, etc.) as those skilled in the art will appreciate.

In order to confirm the activity of PFLF as an adjuvant or auxiliary liver, 10 pieces of PFLF were inserted in the omentum of a rat, of which 90% of liver had been excised. Following this procedure, the rat survived for one month by the functions of auxiliary liver. Generally, a rat with only a 70% hepatotomy shows a temporary decrease in the liver functions, but the liver regenerates to 100% by its natural growth. However, a rat with a 90% excision of its liver typically dies within a week. Rats also die within a week when d-galactosamine is given and at about the 10th day with an allo transplant. Using the product of this invention however, a prolongation of life was achieved in rats whose liver functions were totally impaired or whose liver is almost totally excised. The effect of the implanted product on the omentum tissue was observed and it was found to have almost no effect.

The auxiliary liver product of this invention may be used not only in rats, but also in other mammals, including man. The body weight of a typical rat is 250 gm and of a man is about 60 kg, thus a typical man has about 240 times the body weight of a rat. In the case of rat, 10 sachets containing 10 pieces of PFLF were used. Direct extrapolation to man suggests that a typical man will need over 200 sachets of a equivalent size. For convenience in use, the sachet should be made as large as possible considering the size of the recipients omentum so that the number of sachets may be reduced. When the auxiliary liver product of this invention is used, it is possible to improve its metabolism by inserting it into the omentum rather than in the peritoneal membrane or cavity. For human use, the size of each sachet should be about 60x100 mm, in order to fit in the omentum.

When a total hepatotomy is performed and a whole liver is transplanted, rejection may occur due to the immuno-incompatibility. Unlike such a case the auxiliary liver product of this invention is made from an immature (fetal) pig liver for which blood types have not yet developed, therefore, there is a minimum chance of immuno-reactions due to rejection. PFLF is used in a capsule (sachet) made of semi-permeable membrane and is effective in the case when liver for transplant is not available. This construction also helps to reduce the incidence of rejection.

PFLF is made in the size of 0.5-5.0 mm pieces, preferably in a 1.0-3.0 mm range. If the fragments are made in a size smaller than these, they might lose their function as liver tissue and might not be useful as a supplement for liver. If the size is larger than these, penetration by the DMSO containing preserving solution may be incomplete (the antifreeze solution) and the long term storage of the liver tissue becomes difficult. In the examples, PFLF is made in the shape of cubes, but it also can be made in many other shapes (long, flat, round).

While certain specific embodiments of the invention have been described with particularity herein, it will be recognized that various modifications thereof will occur to those skilled in the art and it is to be understood that such modifications and variations are to be included within the purview of this application and the spirit and scope of the appended claims.

## Claims

1. An auxiliary liver suitable for long term storage while retaining biological activity as liver which comprises liver tissue fragments prepared from the liver of a fetal pig of a gestational age of over 70 days, which are aseptically removed from a pregnant pig and dissected into fragments, treated with an anti-freeze solution and stored at -198°C.

2. The auxiliary liver of claim 1 made from a fetal pig having a gestational age over 90 days.

3. The auxiliary liver of claim 1 made with an anti-freeze solution comprising an aqueous solution containing 5.0-10.0% dimethyl sulfoxide (DMSO) or glycerol and 40.0-60.0% of a phosphate buffer solution.

4. The auxiliary liver of claim 3 wherein the anti-freeze solution also contains a nutrient solution.

5. The auxiliary liver of claim 1 wherein the liver tissue fragments have a size of 0.5-5.0 mm.

6. The auxiliary liver of claim 5 wherein the liver fragments have a size of 1.0-3.0 mm.

7. A process of making an auxiliary liver suitable for a long term storage while retaining biological liver activity comprises:
(a) aseptically removing a fetus having a gestational age of over 70 days from a pregnant pig;
(b) dissecting the fetal liver into fragments;
(c) treating the fetal liver fragments with an anti-freeze solution; and
(d) storing the fragment at -198°C.

8. An auxiliary liver product suitable for implantation in a human comprising liver tissue fragments prepared from the liver of a fetal pig of a gestational age of over 70 days retained in a capsule of a membrane having a selective permeability.

9. The auxiliary liver product of claim 8 made from a fetal pig having a gestational age over 90 days.

10. The auxiliary liver product of claim 8 wherein the liver tissue fragments have a size of 0.5-5.0 mm.

11. The auxiliary liver product of claim 8 wherein the liver fragments have a size of 1.0-3.0 mm.
